# EUROPÄISCHE PATENTSCHRIFT

(11) **EP 0 642 783 B1**
(45) Veröffentlichungstag und Bekanntmachung des Hinweises auf die Patenterteilung: **06.03.1996**
(21) Anmeldenummer: 94113272.2
(22) Anmeldetag: 25.08.1994
(51) Int. Cl.: A61K 7/13

(54) **Mittel zum gleichzeitigen Färben und Aufhellen von menschlichen Haaren**
Composition for simultaneously coloring and lightening human hair
Composition pour la coloration et l'éclairicsement simultanés des cheveux humains

(30) Priorität: 14.09.1993 DE 4331136
(43) Veröffentlichungstag der Anmeldung: 15.03.1995
(73) Patentinhaber: GOLDWELL GmbH, D-64297 Darmstadt (DE)
(72) Erfinder: Lorenz, Heribert, D-64401 Gross-Bieberau (DE); Misu, Daisuke, D-64319 Pfungstadt (DE)

(56) Entgegenhaltungen:
- EP-A- 0 026 473
- EP-A- 0 148 466
- EP-A- 0 545 257
- WO-A-88/01161
- WO-A-88/01162
- DE-A- 2 028 818
- DE-A- 2 222 001
- US-A- 5 182 110
- CHEMICAL ABSTRACTS, vol. 78, no. 14, 9. April 1973, Columbus, Ohio, US; abstract no. 88523, HAYAKAWA MASAKATSU 'Bleaches and dyes for hair'

## Beschreibung

Die vorliegende Erfindung bezieht sich auf ein neues Haarfärbemittel auf Basis von Oxidationsfarbstoff-Vorprodukten, das mindestens eine Enwickler- und mindestens eine Kupplersubstanz enthält und gegenüber herkömmlichen Haarfärbemitteln sowohl eine schnellere Färbung als auch eine Aufhellung des Haares bewirkt.

Konventionelle Haarfärbemittel bestehen in der Regel aus mindestens einer Entwickler- und mindestens einer Kupplersubstanz und enthalten ggf. noch direktziehende Farbstoffe als Nuanceure. Vor ihrer Anwendung auf menschliches Haar werden sie mit verdüunter wäßriger Wasserstoffperoxid-Lösung vermischt. Aus der EP-A 545 257 sind bereits Haarfärbemittel bekannt, die neben Oxidationsfarbstoff-Vorprodukten noch Iodid-Ionen liefernde Verbindungen enthalten und einen alkalischen pH-Wert aufweisen.

Die EP-A 148 466 beschreibt Oxidations-Haarfärbemittel die Ammoniak und wasserlösliche Ammoniumsalze enthalten, einen pH-Wert von 7 bis 9,5 aufweisen und daher haarschauend wirken sollen. Die Einwirkungsdauer auf dem Haar zur Erzielung einer vollständigen Ausfärbung liegt zwischen etwa 30 und 40 Minuten. Es ist naheliegend, daß bei den Benutzern dieser Haarfarben ein Bedürfnis besteht, diese Einwirkungszeit zu verringern.

Gleichzeitig besteht auch vielfach der Wunsch, zusammen mit der Haarfärbung auch eine Aufhellung des zu färbenden Haares, die sich auch als erhöhter Glanz bemerkbar macht, zu erreichen.

Diese Aufgabe wird nun durch die vorliegende Erfindung gelöst, die ein Haarfärbemittel, das mindestens eine Entwickler- und mindestens eine Kupplersubstanz enthält, betrifft, wobei diesem Haarfärbemittel zur Beschleunigung der Oxidationsreaktion bestimmte Katalysatoren zugesetzt werden und gleichzeitig bestimmte Ammoniumverbindungen enthalten sind, und der pH-Wert des gebrauchsfertigen, mit Peroxid vermischten Mittels zwischen 8 und 11, insbesondere 9 und 10, liegt.

Im einzelnen enthält das erfindungsgemäße Haarfärbe- und -aufhellungsmittel etwa 0,001 bis 2,5 Gew.-%, vorzugsweise etwa 0,01 bis 1 Gew.-%, bezogen auf die Gesamtzusammensetzung des Mittels vor dem Peroxidzusatz, mindestens einer Verbindung aus der Gruppe Kupferchlorid (CuCl₂), Kupfersulfat (CuSO₄), Kobaltchlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren essentiellen Bestandteil enthalten die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise etwa 1 bis 5 Gew.-%, bezogen auf die Gesamtzusammensetzung des Mittels (ohne Oxidationsmittel).

Aus der EP-A 435 012 sind bereits Haarfärbemittel bekannt, die eine Substanz, die HCO₃⁻-Ionen bildet, und eine Alkali abspaltende Substanz zur Einstellung eines pH-Wertes zwischen 8,2 und 9,0 in einer Phase, und in einer zweiten Phase eine Wasserstoffperoxid-Lösung mit einem pH-Wert zwischen 2,0 und 4,0 enthalten, wobei der pH-Wert der Mischung beider Phasen zwischen 6,5 und 7,9 liegen soll. Mit diesen Färbemitteln lassen sich die mit den erfindungsgemäßen Färbe- und Aufhellungsmitteln zu erzielenden Effekte nicht erreichen.

Als Entwicklersubstanzen können in den erfindungsgemäßen Zusammensetzungen die für diese Zwecke bekannten und üblichen Oxidationsfarbstoff-Vorprodukte eingesetzt werden.

Als solche seien insbesondere 1,4-Diaminobenzol, 2,5-Diaminotoluol, 4-Aminophenol, 2,5-Diaminobenzylalkohol, 1-Amino-4-[bis(2-hydroxyethyl)-amino]benzol, 3-Methyl-4-aminophenol, Tetraaminopyrimidin, Triaminohydroxypyrimidine, insbesondere 2,5,6-Triamino-4-hydroxypyrimidin, Diaminodihydroxypyrimidine, insbesondere 2,4-Dihydroxy-5,6-diaminopyrimidin und 4,6-Dihydroxy-2,5-diaminopyrimidin, und 2-(2'-Hydroxyethylamino)-5-aminotoluol und die Salze der genannten Entwicklersubstanzen genannt.

Es können selbstverständlich auch andere Entwickler, alleine oder im Gemisch untereinander, eingesetzt werden, beispielsweise das bekannte 5,6-Dihydroxyindol und dessen Derivate.

Der Anteil an Entwickler liegt bei etwa 0,05 bis etwa 5 Gew.-%, vorzugsweise 0,1 bis 3, insbesondere 0,25 bis 1,5 Gew.-%, berechnet auf die Gesamtzusammensetzung des peroxidfreien Mittels und, falls es in Form eines Salzes vorliegt, auf die freie Base bezogen.

Bevorzugte Kupplersubstanzen, die vorzugsweise in einem molaren Verhältnis von Entwicklersubstanz zu Kupplersubstanz zwischen etwa 0,5 : 1 und etwa 2,5 : 1 verwendet werden, sind insbesondere Resorcin, 2-Methylresorcin, 4-Chlorresorcin, 2-Aminophenol, 4-(N-Methyl)aminophenol, 3-Aminophenol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 1,3-Diaminobenzol, 1-Ethoxy-2,4-diaminobenzol, 1-Methoxy-2-amino-4-(2-hydroxyethyl)aminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)benzol.

Es können selbstverständlich auch, zur Erzielung besonderer Farbtöne, Gemische aus verschiedenen Kupplersubstanzen eingesetzt werden, insbesondere solche aus Resorcin oder 2-Methylresorcin mit 2-Aminophenol und/oder 3-Aminophenol, aus Resorcin oder 2-Methylresorcin und 1-Methoxy-2-amino-4-(2'-hydroxyethylamino)benzol, Gemische aus 2-Amino-3-hydroxypyridin mit 5-Amino-2-methylphenol und/oder 1-Methoxy-2-amino-4-(2'-hydroxyethylamino)benzol, oder auch 1,3-Diaminobenzol mit 1,4-Diamino-2-chlorbenzol.
Falls erwünscht, können zur Erzielung bestimmter Farbnuancen auch übliche direktziehende Farbstoffe, beispielsweise die bekannten Arianor-Farbstoffe oder auch Nitrofarbstoffe wie 2-Amino-4,6-dinitrophenol, 2-Amino-6-chlor-4-nitrophenol, 2-Amino-4-nitrophenol etc., sowie auch bekannte Pflanzenfarbstoffe, beispielsweise Henna, in geringen Mengen, d.h. zwischen 0,05 und 1 Gew.-%, enthalten sein. Ausgeschlossen ist die Mitverwendung von 2-Nitro-p-phenylendiamin und 4-Nitro-o-phenylendiamin aufgrund ihrer toxikologischen Bedenklichkeit. Die Gesamtmenge des Farbstoffgemisches im Endprodukt beträgt vorzugsweise etwa 0,2 bis etwa 6,0 Gew.-%, insbesondere etwa 0,5 bis etwa 4 Gew.-%, des Haarfärbemittels.

Zur Herstellung des erfindungsgemäßen Haarfärbemittels werden die Oxidationsfarbstoff-Vorprodukte, d.h. das Gemisch aus Entwicklersubstanz und Kupplersubstanz und ggf. anwesenden direktziehenden Farbstoffen sowie Katalysatoren und Ammoniumverbindungen, in einen geeigneten kosmetischen Träger eingearbeitet. Solche sind insbesondere Emulsionen, d.h. Cremes, oder Gele.

Solche Zusammensetzungen und die in ihnen enthaltenen weiteren Stoffe, insbesondere oberflächenaktive Substanzen, Stabilisatoren, Verdickungsmittel etc. sind einschlägiger Stand der Technik und in verschiedenen Monographien, beispielsweise bei K.Schrader, "Grundlagen und Rezepturen der Kosmetika", 2. Auflage (1989, Hüthig Buchverlag), S. 796 bis 815, beschrieben.

Auf diese und andere Monographien wird daher ausdrücklich Bezug genommen.

Die erfindungsgemäßen Zusammensetzungen werden unmittelbar vor der Anwendung mit peroxidhaltigen Zusammensetzungen, beispielsweise mit jeweils gleichen Anteilen 6prozentiger Wasserstoffperoxid-Lösung, vermischt und auf menschliches Haar aufgebracht, wo sie nach etwa 10- bis 20-minütiger, vorzugsweise etwa 15-minütiger, Einwirkung mit Wasser und einem üblichen Shampoo wieder ausgewaschen werden.

Die an sich bevorzugt vervendeten Wasserstoffperoxid-Zusammensetzungen können durch andere Peroxid-Zubereitungen ersetzt sein, beispielsweise Perborate, Harnstoffperoxid, Melaminperoxid, etc.; jedoch gestaltet sich die Dosierung und Handhabung solcher Zubereitungen, die bis zur Anwendung wasserfrei gehalten werden müssen, umständlicher.

Im folgenden wird die Erfindung anhand von Ausführungsbeispielen illustriert.

### Beispiele

In eine Grundlage aus

| | |
|---|---|
| Cetylstearylalkohol | 12,00 (Gew.-%) |
| Cocosmonoethanolamid | 2,30 |
| Stearinsäuremonoethanolamid | 2,30 |
| Propylenglykolmonostearat | 0,60 |
| Oleylalkoholethoxylat (5 EO) | 5,00 |
| Ölsäure | 2,50 |
| 1,2-Propandiol | 1,00 |
| Natriumlaurylsulfat | 0,50 |
| Komplexbildner (EDTA) | 0,50 |
| Natriumsulfit | 0,50 |
| Ascorbinsäure | 0,30 |
| Eiweißhydrolysat | 1,00 |
| Parfum | 0,40 |
| Ammoniak (25%ig) | 8,00 |
| Wasser | @ 100,00 |

wurden die folgenden, mit den Nummern 1 bis 5 bezeichneten Gemische (jeweils in Gew.-%, bezogen auf die Gesamtzusammensetzung) eingebracht:

| Nr. 1 | |
|---|---|
| 2,5-Diaminotoluolsulfat | 0,75 |
| Resorcin | 0,08 |
| 4-Chlorresorcin | 0,25 |
| 3-Aminophenol | 0,05 |
| 5-Amino-2-methylphenol | 0,02 |
| Calciumchlorid | 0,10 |
| Ammoniumchlorid | 0,50 |

40 g dieser Färbecreme werden mit 40 ml 6%iger wäßriger Wasserstoffperoxid-Lösung vermischt und 15 Minuten bei 30 °C auf Haarsträhnen ausgefärbt.

Nach dem Shampoonieren und Trocknen wird ein intensiv dunkelblond gefärbtes Haar erhalten.

Weglassen des Ammoniumchlorids bei ansonsten gleicher Prozedur ergab eine stumpfere, weniger glänzende Färbung; ein Weglassen des Calciumchlorids führte nach 15 Minuten zu keiner vollständigen Ausfärbung.

| Nr. 2 | |
|---|---|
| 2,5-Diaminotoluolsulfat | 0,75 |
| Resorcin | 0,08 |
| 4-Chlorresorcin | 0,25 |
| 3-Aminophenol | 0,05 |
| 5-Amino-2-methylphenol | 0,02 |
| Hydrochinon | 0,50 |
| Ammoniumhydrogencarbonat | 4,00 |

Die Färbung erfolgte analog der unter Nr. 1 beschriebenen Prozedur, wobei ein hellglänzendes dunkelblondes Haar erhalten wurde.

Weglassen des Ammoniumhydrogencarbonats führte zu einem stumpfen Farbbild; Weglassen des Hydrochinons ergab nach 15 Minuten keine vollständige Ausfärbung.

| Nr. 3 | |
|---|---|
| 2,5-Diaminotoluolsulfat | 0,75 |
| Resorcin | 0,08 |
| 4-Chlorresorcin | 0,25 |
| 3-Aminophenol | 0,05 |
| 5-Amino-2-methylphenol | 0,02 |
| Kaliumjodid | 0,02 |
| Ammoniumchlorid | 3,00 |

Es wurde nach der in Nr. 1 beschriebenen Prozedur verfahren, wobei wiederum eine glänzende Dunkelblond-Färbung erhalten wurde.

Weglassen des Ammoniumchlorids ergab eine stumpfe Farbe; Weglassen des Kaliumjodids eine unvollständige Färbung nach 15 Minuten.

| Nr.4 | |
|---|---|
| 2,5-Diaminotoluolsulfat | 0,54 |
| Resorcin | 0,05 |
| 4-Chlorresorcin | 0,20 |
| 3-Aminophenol | 0,02 |
| 5-Amino-2-methylphenol | 0,01 |
| Kaliumjodid | 0,02 |
| Ammoniumsulfat | 1,00 |

Die Färbung erfolgte nach dem in Nr. 1 beschriebenen Verfahren.
Es wurde ein glänzendes Mittelblond erhalten.

Weglassen des Ammoniumsulfats führte zu einem Verlust des hellen Glanzes; Weglassen des Kaliumjodids ergab keine vollständige Ausfärbung nach 15 Minuten.

| Nr. 5 | |
|---|---|
| 2,5-Diaminotoluolsulfat | 0,90 |
| Resorcin | 0,15 |
| 3-Aminophenol | 0,10 |
| 4-Aminophenol | 0,40 |
| 5-Amino-2-methylphenol | 0,10 |
| Calciumchlorid | 0,30 |
| Ammoniumcarbamat | 2,00 |

Es wurde, wie in Nr. 1 beschrieben, verfahren und eine kräftige, helle haselnußbraune Haarfärbung erzielt.

Weglassen des Ammoniumcarbamats führte zu einem stumpfen Farbton; Weglassen des Calciumchlorids zu einer unvollständigen Färbung nach 15 Minuten.

## Patentansprüche

1. Mittel zum gleichzeitigen Färben und Aufhellen von menschlichen Haaren auf Basis von Oxidationsfarbstoff-Vorprodukten, enthaltend mindestens eine Entwickler- und mindestens eine Kupplersubstanz, dadurch gekennzeichnet, daß es zusätzlich eine Mischung aus
(a) etwa 0,001 bis etwa 2,5 Gew.-%, berechnet auf die Gesamtzusammensetzung (ohne Oxidationsmittel), mindestens einer der Verbindungen Kupferchlorid, Kupfersulfat, Kaliumjodid, Natriumjodid, Calciumchlorid, Calciumnitrat, Lithiumchlorid, Magnesiumacetat, Kaliumbichromat, Bariumnitrat, Kobaltchlorid, Cersulfat, Cerchlorid, Vanadiumsulfat, Mangandioxid und/oder Hydrochinon, und
(b) etwa 0,5 bis etwa 10 Gew.-%, berechnet auf die Färbemittelzusammensetzung (ohne Oxidationsmittel), mindestens einer Ammoniumverbindung, ausgewählt aus der Gruppe Ammoniumchlorid, Ammoniumsulfat, Ammoniumcarbonat, Ammoniumbicarbonat und/oder Ammoniumcarbamat enthält, und
(c) nach dem Vermischen mit Peroxid einen pH-Wert zwischen 8 und 11 aufweist.

2. Mittel nach Anspruch 1, dadurch gekennzeichnet, daß es etwa 0,01 bis etwa 1 Gew.-% mindestens einer der unter (a) aufgeführten Substanzen enthält.

3. Mittel nach Anspruch 1 und/oder 2, dadurch gekennzeichnet, daß es etwa 1 bis 5 Gew.-% einer der unter (b) bezeichneten Ammoniumverbindungen enthält.

4. Mittel nach einem oder mehreren der Ansprüche 1 bis 3, dadurch gekennzeichnet, daß es nach dem Vermischen mit Peroxid einen pH-Wert zwischen 9 und 10 aufweist.

## Claims

1. Composition for simultaneous dyeing and brightening of human hair, comprising at least one developing and at least one coupling agent, characterized in that it additionally contains a mixture of
(a) about 0.001 to about 2.5% by wt., calculated to the total composition (excluding oxidizing agent), of at least one of the compounds copper chloride, copper sulfate, potassium iodide, sodium iodide, calcium chloride, calcium nitrate, lithium chloride, magnesium acetate, potassium bichromate, barium nitrate, cobalt chloride, cerium sulfate, cerium chloride, vanadium sulfate, manganese dioxide, and/or hydroquinone, and
(b) about 0.5 to about 10% by wt., calculated to the dyeing composition (excluding oxidizing agent), of at least one ammonium compound selected from the group ammonium chloride, ammonium sulfate, ammonium carbonate, ammonium bicarbonate, and (or) ammonium carbamate, and
(c) having a pH-value between 8.0 and 11 after admixture with peroxide.

2. Composition according to claim 1, characterized in that it comprises about 0.01 to about 1% by wt. of at least one of the substances listed in (a).

3. Composition according to claim 1 and (or) 2, characterized in that it contains about 1 to 5% by wt. of one of the ammonium compounds listed in (b).

4. Composition according to one or more of the claims 1 to 3, characterized in that it has a pH-value between 9 and 10 after admixture with peroxide.

## Revendications

1. Composition pour la coloration et l'éclaircissement simultanés des cheveux humains, à base de précurseurs de colorant d'oxydation, contenant au moins un révélateur et au moins un coupleur, caractérisée en ce qu'elle contient en outre un mélange de :
a) environ 0,001 à environ 2,5% en poids, calculé sur la base de la composition totale (sans oxydant), d'au moins un composé parmi le chlorure de cuivre, le sulfate de cuivre, l'iodure de potassium, l'iodure de sodium, le chlorure de calcium, le nitrate de calcium, le chlorure de lithium, l'acétate de magnésium, le bichromate de potassium, le nitrate de baryum, le chlorure de cobalt, le sulfate de cérium, le chlorure de cérium, le sulfate de vanadium, le dioxyde de manganèse et/ou l' hydroquinone, et
b) environ 0,5 à environ 10% en poids, calculé sur la base de la composition de colorants (sans oxydant), d'au moins un composé d'ammonium choisi parmi le chlorure d'ammonium, le sulfate d'ammonium, le carbonate d'ammonium, le bicarbonate d'ammonium et/ou le carbamate d'ammonium, et
c) après mélange avec un peroxyde, elle présente un pH entre 8 et 11.

2. Composition selon la revendication 1, caractérisée en ce qu'elle contient environ 0,01 à environ 1% en poids d'au moins une des substances indiquées en a).

3. Composition selon la revendication 1 et/ou 2, caractérisée en ce qu'elle contient environ 1 à 5% en poids d'un des composés d'ammomium décrits en b).

4. Composition selon l'une ou plusieurs des revendications 1 à 3, caractérisée en ce qu'elle présente un pH entre 9 et 10 après mélange avec un peroxyde.
